# EUROPEAN PATENT SPECIFICATION

(11) **EP 0 832 900 B1**
(45) Date of publication and mention of the grant of the patent: **03.03.2004**
(21) Application number: 96915261.0
(22) Date of filing: 06.05.1996
(51) Int. Cl.: C07K 5/06, C07K 5/08, C07K 5/10, A61K 38/05, A61K 38/06, A61K 38/07

(54) **PEPTIDE AND METHOD OF OBTAINING IT**
PEPTIDE UND GEWINNUNGSVERFAHREN
PEPTIDE ET PROCEDE DE PRODUCTION

(30) Priority: 07.06.1995 RU 95108559
(43) Date of publication of application: 01.04.1998
(73) Proprietor: Immunotech Developments Inc., Mississauga, Ontario L5K 1B3 (CA)
(72) Inventor: Deigin, Vladislav Isakovich, Moscow, 121609 (RU); Korotkov, Andrei Marxovich, Moscow, 121609 (RU)
(74) Representative: Benedum, Ulrich Max, Dr.
(86) International application number: PCT/RU1996/000116
(87) International publication number: WO 1996/040740

(56) References cited:
- EP-A- 0 101 929
- WO-A-89/06134
- WO-A-95/03067
- GB-A- 1 526 367
- GB-A- 2 109 796
- US-A- 3 832 337

## Description

The invention relates to medicine, namely to methods for preparing biologically active substances possessing immunoregulating properties, and can be used in medicine, veterinary medicine and experimental biochemistry.

### The Prior Art

The importance of developing new harmless immunoregulating peptides, which would stop the growing incidence of such diseases as malignant neoplasms, sepsis, chronic and latent infections progressing against the background of immunodeficiencies, is proved by the great number of studies in this field.

The commonest method for isolation of new peptides consists of isolation of active peptide fractions from tissue extracts, fractionation and purification including isolation of individual substances and their identification (SU N1737798, inventor's certificate, granted on March 20, 1995). In practical medicine, thymic extracts are widely known as regulators of immuno processes, for instance, thymosin fraction 5 (Goldstein, A.L., Guna A., Latz M.M., Hardy H.A., White A.) thyamalin (CH, N 6595 86). The extracts contain substances of polypeptides nature, their production from natural raw material is limited by the complicity of the process, small yields of active substances, and a considerable variability of their physical and chemical characteristics and biological properties. Besides, the ballast components present in natural thymic preparations sometimes cause side effects. The last circumstance became a stimulus for creating synthetic peptides. By now, several immunoregulating peptides have been synthesized: PCT/WO 089/06134, EP-A 230052, EPA 406931, US 5021551, US 5013723. Each of the synthetic peptides has a limited combination of necessary properties, is highly active, low toxic, causes no side effects, and therefore can be used in medicine.

### The Disclosure of the Invention

The object of the claimed invention was to synthesize a new biologically active peptide possessing immunoregulating effect and having the following formula I.

X-A-D-Trp-Y (I)

wherein X is selected from the group consisting of hydrogen, glycine, alanine, leucine, isoleucine, valine, N-valine, proline, tyrosine, phenylalanine, tryptophan, D-alanine, D-leucine, D-isoleucine, D-valine, D-N-valine, D-proline, D-tyrosine, D-phenylalanine, D-tryptophan, γ-aminobutyric acid, and ξ-aminocaproic acid; A is selected from the group consisting of D-glutamic acid and iD-glutamic acid; and Y is selected from the group consisting of glycine, alanine, leucine, isoleucine, valine, N-valine, proline, tyrosine, phenylalanine, tryptophan, D-alanine, D-leucine, D-isoleucine, D-valine, D-N-valine, D-proline, D-tyrosine, D-phenylalanine, D-tryptophan, γ-aminobutyric acid, ξ-aminocaproic acid, hydroxyl and C₁ to C₃ substituted amide.

Another object of the invention was to develop a fundamentally new technology of peptide synthesis, which would have minimum simple stages and provide high yield of the product of the above mentioned structure, which is decisive in pharmaceutical production.

The present invention therefore also relates to a method of preparing a peptide of the formula I

X-A-D-Trp-Y (I)

wherein X, A and Y are as defined as above, comprising: synthesizing the peptide in solution by opening the internal anhydride of *tert*.-butyloxycarbonyl glutamine (D or L) acid using K-salt of D-Trp-Y; further chain building with the use of activated ethers and mixed anhydrides, with preliminary detachment of *tert*.-butyloxycarbonyl group by treating with formic acid, and with the following chromatographic separation of α- and γ-isomers.

The principle of the new method consists in synthesizing glutamyl-containing peptides in solution by opening the internal anhydride of tert.-butyl oxycarbonyl glutamine (D or L) acid using the respective D-Trp-Y derivative, with the following chromatographic separation of α- and γ- isomers. The further synthesis was carried out by building the peptide chain with the use of activated ethers of tretbutyl oxycarbonyl amino acids.

Table 1 presents Rf₁ (in chloroform-methanol-32% acetic acid = 60:45:20) and Rf₂ (in butanol-pyridine-water-acetic acid = 5:5:4:1) values for a number of analogs of the new peptide.

| Peptide | R_{f1} | R_{f2} |
|---|---|---|
| Abu-D-Glu-D-Trp-OH | 0.32 | 0.53 |
| Abu-iD-Glu-D-Trp-OH | 0.29 | 0.49 |
| Aca-D-Glu-D-Trp-OH | 0.31 | 0.52 |
| Aca-iD-Glu-D-Trp-OH | 0.28 | 0.49 |
| Ala-D-Glu-D-Trp-OH | 0.34 | 0.61 |
| Ala-iD-Glu-D-Trp-OH | 0.32 | 0.55 |
| D-Ala-D-Glu-D-Trp-D-Ala | 0.22 | 0.41 |
| D-Ala-D-Glu-D-Trp-OH | 0.26 | 0.51 |
| D-Ala-iD-Glu-D-Trp-D-Ala | 0.25 | 0.50 |
| D-Ala-iD-Glu-D-Trp-OH | 0.25 | 0.51 |
| D-Ile-D-Glu-D-Trp-D-Leu | 0.35 | 0.53 |
| D-Ile-D-Glu-D-Trp-OH | 0.36 | 0.54 |
| D-Ile-iD-Glu-D-Trp-D-Leu | 0.34 | 0.53 |
| D-Ile-iD-Glu-D-Trp-OH | 0.27 | 0.52 |
| D-Leu-D-Glu-D-Trp-OH | 0.36 | 0.53 |
| D-Leu-iD-Glu-D-Trp-OH | 0.35 | 0.52 |
| D-NVal-D-Glu-D-Trp-OH | 0.33 | 0.51 |
| D-NVal-iD-Glu-D-Trp-OH | 0.32 | 0.51 |
| D-Phe-D-Glu-D-Trp-Ala | 0.37 | 0.56 |
| D-Phe-iD-Glu-D-Trp-Ala | 0.36 | 0.55 |
| D-Pro-D-Glu-D-Trp-OH | 0.38 | 0.58 |
| D-Pro-iD-Glu-D-Trp-OH | 0.37 | 0.58 |
| D-Trp-D-Glu-D-Trp-OH | 0.41 | 0.59 |
| D-Trp-iD-Glu-D-Trp-OH | 0.40 | 0.59 |
| D-Tyr-D-Glu-D-Trp-D-Tyr | 0.39 | 0.58 |
| D-Tyr-iD-Glu-D-Trp-D-Tyr | 0.38 | 0.58 |
| D-Val-D-Glu-D-Trp-OH | 0.34 | 0.51 |
| D-Val-iD-Glu-D-Trp-OH | 0.33 | 0.50 |
| Gly-D-Glu-D-Trp-NVal | 0.29 | 0.51 |
| Gly-D-Glu-D-Trp-OH | 0.30 | 0.54 |
| Gly-iD-Glu-D-Trp-NVal | 0.32 | 0.53 |
| Gly-iD-Glu-D-Trp-OH | 0.29 | 0.53 |
| H-D-Glu-D-Trp-Abu | 0.33 | 0.52 |
| H-D-Glu-D-Trp-Aca | 0.30 | 0.53 |
| H-D-Glu-D-Trp-D-Ile | 0.35 | 0.58 |
| H-D-Glu-D-Trp-D-Pro | 0.37 | 0.61 |
| H-D-Glu-D-Trp-Gly | 0.29 | 0.54 |
| H-D-Glu-D-Trp-Ile | 0.36 | 0.58 |
| H-D-Glu-D-Trp-Leu | 0.36 | 0.59 |
| H-D-Glu-D-Trp-Lys | 0.28 | 0.47 |
| H-D-Glu-D-Trp-OH | 0.36 | 0.56 |
| H-D-Glu-D-Trp-Phe | 0.39 | 0.61 |
| H-D-Glu-D-Trp-Pro | 0.38 | 0.59 |
| H-D-Glu-D-Trp-Tyr | 0.37 | 0.58 |
| H-D-Glu-D-Trp-Val | 0.38 | 0.61 |
| H-iD-Glu-D-Trp-Abu | 0.34 | 0.51 |
| H-iD-Glu-D-Trp-Aca | 0.33 | 0.50 |
| H-iD-Glu-D-Trp-D-Ile | 0.34 | 0.55 |
| H-iD-Glu-D-Trp-D-Pro | 0.36 | 0.58 |
| H-iD-Glu-D-Trp-Gly | 0.28 | 0.52 |
| H-iD-Glu-D-Trp-Ile | 0.34 | 0.57 |
| H-iD-Glu-D-Trp-Leu | 0.35 | 0.58 |
| H-iD-Glu-D-Trp-Lys | 0.27 | 0.45 |
| H-iD-Glu-D-Trp-OH | 0.30 | 0.52 |
| H-iD-Glu-D-Trp-Phe | 0.38 | 0.61 |
| H-iD-Glu-D-Trp-Pro | 0.37 | 0.60 |
| H-iD-Glu-D-Trp-Tyr | 0.35 | 0.58 |
| H-iD-Glu-D-Trp-Val | 0.36 | 0.60 |
| Ile-D-Glu-D-Trp-D-Phe | 0.42 | 0.68 |
| Ile-D-Glu-D-Trp-OH | 0.38 | 0.56 |
| Ile-iD-Glu-D-Trp-D-Phe | 0.40 | 0.67 |
| Ile-iD-Glu-D-Trp-OH | 0.37 | 0.55 |
| Leu-D-Glu-D-Trp-OH | 0.35 | 0.62 |
| Leu-iD-Glu-D-Trp-OH | 0.27 | 0.57 |
| NVal-D-Glu-D-Trp-OH | 0.35 | 0.61 |
| NVal-iD-Glu-D-Trp-OH | 0.34 | 0.60 |
| Phe-D-Glu-D-Trp-OH | 0.41 | 0.63 |
| Phe-iD-Glu-D-Trp-OH | 0.40 | 0.62 |
| Pro-D-Glu-D-Trp-OH | 0.43 | 0.63 |
| Pro-iD-Glu-D-Trp-OH | 0.42 | 0.62 |
| Trp-D-Glu-D-Trp-OH | 0.45 | 0.68 |
| Trp-iD-Glu-D-Trp-OH | 0.44 | 0.67 |
| Tyr-D-Glu-D-Trp-OH | 0.42 | 0.64 |
| Tyr-iD-Glu-D-Trp-OH | 0.41 | 0.63 |
| Val-D-Glu-D-Trp-D-Val | 0.46 | 0.68 |
| Val-D-Glu-D-Trp-OH | 0.33 | 0.50 |
| Val-iD-Glu-D-Trp-D-Val | 0.44 | 0.66 |
| Val-iD-Glu-D-Trp-OH | 0.34 | 0.52 |

### The Preferred Embodiments of the Invention

The following example is given to illustrate the invention.

Example.
H-D-Glu-D-Trp-OH and H-iD-Glu-D-Trp-OH

### 1. Preparation of Boc-D-Glu-OH

14.7 g (0.1 M) of H-D-Glu-OH were dissolved in 200 ml distilled water. pH was adjusted to 10.2 with 1 M KOH and a solution of 33.0 g (0.3 M) BOC₂) in dioxane was added with intensive mixing. The pH was monitored using a pH-stat. On completion of the reaction, the mixture was transferred into a separating funnel, extraction from alkaline medium with 3 x 150 ml ethyl acetate was carried out 0.2% sulfuric acid solution was slowly added to the water phase to adjust pH to 3.0, and Boc-D-Glu-OH was extracted into the organic phase (3 x 200 ml). The organic layer was washed with 3 x 200 ml Na₂SO₄ saturated solution to neutral pH, dried, over Na₂SO₄, then evaporated to oil under vacuum. Yield: 16.7 g (68%).

### 2. Preparation of the mixture of Boc-D-Glu-Trp-OH and Boc-iD-Glu-D-Trp-OH

16.7 g (0.068 M) Boc-D-Glu-OH were dissolved in 200 ml and dimethyl formamide, cooled to 0°C and to this was added the solution of 20.6 g (0.1 M) N, N'-dicyclohexyl carbodiimide in 100 ml dimethyl formamide with mixing. The mixture was stirred at +4 °C for 4 h, and then it was allowed to stay 8 h at room temperature. The precipitated dicyclohexyl carbamide was separated by filtration and washed with 2 x 50 ml dimethyl formamide. The filtrate was concentrated by evaporation under vacuum to 1/2 of the volume and to this were added 24.3 g (0.1 M) H-D-Trp-OH with cooling to +4 °C and vigorous mixing. The solution was allowed to warm up to room temperature. The completion of the reaction was controlled by TLC in the system of chloroform: ethyl acetate: methanol= 6:3:1 by disappearance of the spot of the internal anhydride of Boc-D-Glu acid. The rest of dihexyl carbamide was separated by filtration, dimethyl formamide was evaporated under vacuum. 200 ml ethyl acetate and 200 ml 0.2% sulfuric acid solution were added to the oil-like residue. The organic layer was separated, rinsed to neutral pH with Na₂SO₄ saturated solution, dried over Na₂SO₄. The ethyl acetate solution was evaporated under vacuum. The resultant oil-like precipitate was a mixture of Boc-D-Glu-D-Trp-OH and Boc-iD-Glu-D-Trp-OH. Total yield: 25.4 g (70%).

### 3. Preparation of H-D-Glu-D-Trp-OH and H-iD-Glu-D-Trp-OH.

25.4 g of the mixture (0.048 M) were dissolved in 200 ml of formic acid, and stirred for 1 h at 40°C. The solvent was evaporated under vacuum to thick oil. The peptides were separated and purified by ion exchange chromatography in a Sephadex® column in a gradient of 0.01 - 0.2 M pyridine acetate buffer. Yield: 5.7 g (35%) of H-D-Glu-D-Trp-OH and 5.7 g (35%) of H-iD-Glu-D-Trp-OH.

The following physical and chemical characteristics of the peptide were found:
Primary structure -H-D-Glu-D-Trp-OH and H-iD-Glu-D-Trp-OH.
Empirical formula -C₁₆H₂₀N₃O₅
Molecular weight- 334.35
Appearance-yellowish-white or grey powder
Solubility- readily soluble in water, moderately soluble in alcohol, insoluble in chloroform.
The UV - spectrum in the range of 250-300 nm exhibits a maximum at 280 ± 2 nm and a shoulder at 287 ± 2 nm.

The biological activity of the new peptide was studied in Balb/c mice. Splenic cells of mice were suspended in the RPMI 1640 medium with 2 ml of glutamine and 5% inactivated fetal serum, and then dispensed into flat-bottomed plates: 100 mcl (200,000 cells) per well. The preparation under study was added at the beginning of cultivation. As a mitogen, concanavallin A was used in the final concentration of 2 µg per well. The plates were incubated at 37°C and in 5% CO₂ for 48 h. Proliferation of the cells was assessed by incorporation of 3H-thymidine, which was added in the dose of 5 µg/ml 24 h before the end of cultivation, with the help of a scintillation counter and was expressed in counts per minute (CPM). The results are presented in Table 2.

**Table 2**

| Preparation | CPMI* | | | |
|---|---|---|---|---|
| | Preparation dose (µg/ml) | | | |
| | 1 | 5 | 10 | 20 |
| iD-Glu-D-Trp | 68594 | 63428 | 20043 | 13222 |
| Cyclosporin A | 67649 | 2698 | 574 | 569 |
| Control | 61467 | | | |

| | | | | |
|---|---|---|---|---|
| * - mean value of three measurements | | | | |

The new peptide was found to inhibit proliferation of splenic cells.

To study the safety of the peptide, an acute toxicity study was carried out in compliance with Methodical Recommendations of the Pharmacological Committee of the RF "Requirements to Preclinical Studies of General Toxic Effect of New Pharmacological Substances". M., 1985.

According to the results obtained, a 1000-fold intraperitoneal dose of the peptide caused no acute toxic effect, and it was impossible to achieve LD₅₀ with these doses.

### Commercial Application

The biologically active peptide may be widely used in medicine and veterinary medicine.

## Claims

1. A peptide of the formula I
X-A-D-Trp-Y (I)
wherein X is selected from the group consisting of hydrogen, glycine, alanine, leucine, isoleucine, valine, N-valine, proline, tyrosine, phenylalanine, tryptophan, D-alanine, D-leucine, D-isoleucine, D-valine, D-N-valine, D-proline, D-tyrosine, D-phenylalanine, D-tryptophan, γ-aminobutyric acid, and ξ-aminocaproic acid; A is selected from the group consisting of D-glutamic acid and iD-glutamic acid; and Y is selected from the group consisting of glycine, alanine, leucine, isoleucine, valine, N-valine, proline, tyrosine, phenylalanine, tryptophan, D-alanine, D-leucine, D-isoleucine, D-valine, D-N-valine, D-proline, D-tyrosine, D-phenylalanine, D-tryptophan, γ-aminobutyric acid, ξ-aminocaproic acid, hydroxyl and C₁ to C₃ substituted amide.

2. A peptide having the sequence H-iD-Glu-D-Trp-OH.

3. A pharmaceutical composition comprising at least one of the peptides of the formula I as claimed in claims 1 or 2 and a pharmaceutically acceptable vehicle.

4. A use of the peptide as defined in claims 1 or 2 for inhibiting splenic cell proliferation.

5. A use of the peptide as defined in claims 1 or 2 for its immunoregulating effect.

6. A method for preparing a peptide of the formula I
X-A-D-Trp-Y (I)
wherein X is selected from the group consisting of hydrogen, glycine, alanine, leucine, isoleucine, valine, N-valine, proline, tyrosine, phenylalanine, tryptophan, D-alanine, D-leucine, D-isoleucine, D-valine, D-N-valine, D-proline, D-tyrosine, D-phenylalanine, D-tryptophan, γ-aminobutyric acid, and ξ-aminocaproic acid; A is selected from the group consisting of D-glutamic acid and iD-glutamic acid; and Y is selected from the group consisting of glycine, alanine, leucine, isoleucine, valine, N-valine, proline, tyrosine, phenylalanine, tryptophan, D-alanine, D-leucine, D-isoleucine, D-valine, D-N-valine, D-proline, D-tyrosine, D-phenylalanine, D-tryptophan, γ-aminobutyric acid, ξ-aminocaproic acid, hydroxyl and C₁ to C₃ substituted amide, comprising:
synthesizing the peptide in solution by opening the internal anhydride of tert-butyloxycarbonyl glutamine (D or L) acid using K-salt of D-Trp-Y; further chain building with the use of activated ethers and mixed anhydrides, with preliminary detachment of tert-butyloxycarbonyl group by treating with formic acid, and with the following chromatographic separation of α- and γ-isomers.

## Patentansprüche

1. Peptid der Formel I
X-A-D-Trp-Y (I)
wobei X ausgewählt ist aus der Gruppe Wasserstoff, Glycin, Alanin, Leucin, Isoleucin, Valin, N-Valin, Prolin, Tyrosin, Phenylalanin, Tryptophan, D-Alanin, D-Leucin, D-Isoleucin, D-Valin, D-N-Valin, D-Prolin, D-Tyrosin, D-Phenylalanin, D-Tryptophan, γ-Aminobutansäure und ξ-Aminocapronsäure; A ausgewählt aus der Gruppe D-Glutaminsäure und iD-Glutaminsäure, und Y ausgewählt aus der Gruppe Glycin, Alanin, Leucin, Isoleucin, Valin, N-Valin, Prolin, Tyrosin, Phenylalanin, Tryptophan, D-Alanin, D-Leucin, D-Isoleucin, D-Valin, D-N-Valin, D-Prolin, D-Tyrosin, D-Phenylalanin, D-Tryptophan, γ-Aminobutansäure, ξ-Aminocapronsäure, Hydroxyl und C₁- bis C₃-substituierte Amide.

2. Peptid mit der Sequenz H-iD-Glu-D-Trp-OH.

3. Pharmazeutische Zusammensetzung, umfassend mindestens ein Peptid der Formel I, wie in Anspruch 1 oder 2 beansprucht, und einen pharmazeutisch akzeptabler Träger.

4. Verwendung des Peptids nach Anspruch 1 oder 2 zur Inhibierung der Milzzellen-Vermehrung.

5. Verwendung des Peptids nach Anspruch 1 oder 2 wegen seiner immunoregulatorischen Wirkung.

6. Verfahren zur Herstellung eines Peptids nach Formel I
X-A-D-Trp-Y (I)
wobei X ausgewählt ist aus der Gruppe Wasserstoff, Glycin, Alanin, Leucin, Isoleucin, Valin, N-Valin, Prolin, Tyrosin, Phenylalanin, Tryptophan, D-Alanin, D-Leucin, D-Isoleucin, D-Valin, D-N-Valin, D-Prolin, D-Tyrosin, D-Phenylalanin, D-Tryptophan, γ-Aminobutansäure und ξ-Aminocapronsäure; A ausgewählt aus der Gruppe D-Glutaminsäure, iD-Glutaminsäure; und Y ausgewählt aus der Gruppe Glycin, Alanin, Leucin, Isoleucin, Valin, N-Valin, Prolin, Tyrosin, Phenylalanin, Tryptophan, D-Alanin, D-Leucin, D-Isoleucin, D-Isoleucin, D-Valin, N-Valin, D-Prolin, D-Tyrosin, D-Phenylalanin, D-Tryptophan, γ-Aminobutansäure, ξ-Aminocapronsäure, Hydroxyl und C₁- bis C₃-substituierte Amide, umfassend
das Synthetisieren des Peptids in Lösung durch Öffnen des inneren Anhydrids der tert-Butyloxycarbonylglutamin-(D oder L)-Säure mit dem K-Salz von D-Trp-Y, weitere Kettenverlängerung mithilfe von aktivierten Estern und gemischten Anhydriden unter vorläufiger Abtrennung der *tert*-Butyloxycarbonylgruppe durch eine Behandlung mit Ameisensäure und die anschließende chromatographische Auftrennung der α- und γ-Isomere.

## Revendications

1. Peptide de formule I :
X-A-D-Trp-Y (I)
dans laquelle X est choisi dans le groupe consistant en hydrogène, glycine, alanine, leucine, isoleucine, valine, N-valine, proline, tyrosine, phénylalanine, tryptophane, D-alanine, D-leucine, D-isoleucine, D-valine, D-N-valine, D-proline, D-tyrosine, D-phénylalanine, D-tryptophane, acide γ-aminobutyrique et acide ξ-aminocaproïque ; A est choisi dans le groupe consistant en acide D-glutamique et acide iD-glutamique ; et Y est choisi dans le groupe consistant en glycine, alanine, leucine, isoleucine, valine, N-valine, proline, tyrosine, phénylalanine, tryptophane, D-alanine, D-leucine, D-isoleucine, D-valine, D,N-valine, D-proline, D-tyrosine, D-phénylalanine, D-tryptophane, acide γ-aminobutyrique, acide ξ-aminocaproïque, hydroxy et amide substitué en C₁ à C₃.

2. Peptide ayant la séquence H-iD-Glu-D-Trp-OH.

3. Composition pharmaceutique comprenant au moins l'un des peptides de formule 1 selon les revendications 1 ou 2 et un véhicule acceptable du point de vue pharmaceutique.

4. Utilisation du peptide selon les revendications 1 ou 2 pour inhiber la prolifération des cellules spléniques.

5. Utilisation du peptide selon les revendications 1 ou 2 pour son effet immunorégulateur.

6. Procédé de préparation d'un peptide de formule I :
X-A-D-Trp-Y (I)
dans laquelle X est choisi dans le groupe consistant en hydrogène, glycine, alanine, leucine, isoleucine, valine, N-valine, proline, tyrosine, phénylalanine, tryptophane, D-alanine, D-leucine, D-isoleucine, D-valine, D-N-valine, D-proline, D-tyrosine, D-phénylalanine, D-tryptophane, acide γ-aminobutyrique et acide ξ-aminocaproïque ; A est choisi dans le groupe consistant en acide D-glutamique et acide iD-glutamique ; et Y est choisi dans le groupe consistant en glycine, alanine, leucine, isoleucine, valine, N-valine, proline, tyrosine, phénylalanine, tryptophane, D-alanine, D-leucine, D-isoleucine, D-valine, D,N-valine, D-proline, D-tyrosine, D-phénylalanine, D-tryptophane, acide γ-aminobutyrique, acide ξ-aminocaproïque, hydroxy et amide substitué en C₁ à C₃ :
comprenant :
la synthèse du peptide en solution par ouverture de l'anhydride interne d'acide t-butyloxycarbonyl glutamine (D) ou (L) avec un sel de potassium de D-Trp-Y ;
la poursuite de la construction de la chaîne avec des éthers activés et des anhydrides mixtes, avec le détachement préliminaire du groupe t-butyloxycarbonyle par traitement avec de l'acide formique, et avec la séparation chromatographique ultérieure des isomères α et γ.
